# EUROPEAN PATENT SPECIFICATION

(11) **EP 3 222 748 B1**
(45) Date of publication and mention of the grant of the patent: **20.10.2021**
(21) Application number: 16858775.6
(22) Date of filing: 26.07.2016
(51) Int. Cl.: C23C 16/14, C23C 16/02, C23C 16/04, C23C 16/448, A61F 2/30, A61L 27/42, A61L 27/56, A61F 2/28, A61L 27/30

(54) **MEDICAL POROUS TANTALUM METAL MATERIAL AND PREPARATION METHOD THEREFOR**
MEDIZINISCHES PORÖSES TANTALMETALLMATERIAL UND HERSTELLUNGSVERFAHREN DAFÜR
MATÉRIAU MÉTALLIQUE DE TANTALE POREUX À USAGE MÉDICAL ET SON PROCÉDÉ DE PRÉPARATION

(30) Priority: 28.10.2015 CN 201510712170
(43) Date of publication of application: 27.09.2017
(73) Proprietor: Zhao, Dewei, Dalian, Liaoning 116001 (CN); Wang, Benjie, Dalian, Liaoning 116001 (CN); Xie, Hui, Dalian, Liaoning 116001 (CN); Huang, Shibo, Dalian, Liaoning 116001 (CN); Xiaowei, Wei, Dalian, Liaoning 116001 (CN); Wang, Wei, Dalian, Liaoning 116001 (CN)
(72) Inventor: Zhao, Dewei, Dalian, Liaoning 116001 (CN); Wang, Benjie, Dalian, Liaoning 116001 (CN); Xie, Hui, Dalian, Liaoning 116001 (CN); Huang, Shibo, Dalian, Liaoning 116001 (CN); Xiaowei, Wei, Dalian, Liaoning 116001 (CN); Wang, Wei, Dalian, Liaoning 116001 (CN)
(74) Representative: Proi World Intellectual Property GmbH
(86) International application number: PCT/CN2016/091732
(87) International publication number: WO 2017/071336

(56) References cited:
- CN-A- 103 480 043
- CN-A- 103 480 043
- CN-A- 104 338 184
- CN-A- 105 177 523
- P. GONZÁLEZ ET AL: "A new generation of bio-derived ceramic materials for medical applications", JOURNAL OF BIOMEDICAL MATERIALS RESEARCH. PART A, vol. 88A, no. 3, 1 March 2009 (2009-03-01) , pages 807-813, XP055474213, HOBOKEN, NY, US ISSN: 1549-3296, DOI: 10.1002/jbm.a.31951
- WANG, JING ET AL.: 'Porous Biological Materials' POROUS BIOLOGICAL MATERIALS 31 May 2012, XP009503318

## Description

### Technical Field

The present invention relates to a medical porous tantalum metal material and a preparation method thereof, and more particularly, to a medical porous tantalum metal material capable of being applied in an orthopaedic bearing part and a preparation method thereof.

### Background Art

Bone defect repair after bone trauma and bone necrosis has problems such as poor mechanical and osteoinductive properties. After the importance of porous materials in the body is revealed , this problem is expected to be better solved. It is found that the porosity and pore size of the porous material are important factors to determine the success of the implant. Increasing the porosity or reducing the dead space will be beneficial to the bone ingrowth. It is generally believed that the porosity is greater than 60% and the pore diameter is greater than 150µm, which will be beneficial to the bone ingrowth. When the pore size is 200µm-400µm, it is most beneficial to new bone growth. At the same time, the larger porosity can reduce the weight of the implant material and allow it to be close to human bones in biomechanical indexes. But higher requirements shall be met for the processing of materials. To prepare the medical implant materials having high porosity, regular and uniform shape of the pore and high pore connectivity is the goal of research and development.

Tantalum is a metal having a high melting point (2996°C) and corrosion resistance, which has been widely used in chemical industry, metallurgy, aerospace and other fields. In addition, tantalum, which is not reacted with body fluids and does not simulate the body tissue, also has become an ideal material for the preparation of surgical implants. The application in this aspect has been in use for more than half a century, including cardiac pacemakers, repair of skull defects, vascular clamps, femoral stem prosthesis, and metal wires, sheets or meshes for nerve repair, and the like. Since the reduction of tantalum chloride with hydrogen in 1907 to obtain metal tantalum, chemical vapor deposition (CVD) tantalum has been concerned. The CVD method may be used for depositing metal tantalum on components and parts made of metal or non-metallic materials. Because of its good biocompatibility and stability, porous tantalum metal is expected to play an important role in reconstructing bones in a weight-bearing area in vivo.

However, due to the high melting point of tantalum, the traditional loose powder sintering method or the like are more difficult to process, the porosity of the material prepared is lower, the pore size is not uniform and the closed porosity is high. The chemical vapor deposition method is a feasible method to prepare the porous tantalum metal, but tantalum coating usually has a limited thickness, which reduces the mechanical properties of the product; moreover, this method has high requirements on the physical and biological properties of the framework material, that is, the framework material shall have high temperature resistance, mechanical properties suitable for implant environment and no cytotoxicity.

The porous silicon carbide support material adopted in the present invention has the condition of preparing the support of the porous tantalum metal material prepared by applying the chemical vapor deposition method; the porous silicon carbide support material can be processed into irregular shape suitable for in vivo application before plating tantalum, which reduces the possibility of damage to the pores due to post processing. At the same time, the tantalum metal coating is compacted with particle size of 1∼5µm and purity of over 99.5%, and has a sufficient thickness by controlling parameters such as reaction gas concentration, deposition temperature and reaction time during the chemical vapor deposition process, so as to obtain the sufficient mechanical strength of the material.

CN 104 338 184 A relates to medical implantation porous material that comprises braided fabric of a metal wire or a non-metal wire and a metal layer combined on the braided fabric.

CN 103 480 043 A relates to a porous medical tantalum implant material and a preparation method and application thereof.

### Summary of the Invention

An object of the present invention is to provide a medical porous tantalum metal material according to independent claim 1, which is close to the mechanical properties of the human skeleton and can be applied as a permanent implant material to weight-bearing bone or non-weight-bearing parts in human body and a preparation method thereof.

Further, the tantalum metal compound may be of powder with a particle size of 400-mesh.

The present invention further provides a preparation method according to independent claim 3 for the medical porous tantalum metal material. The preparation method comprises the following steps of:
(1) ultrasonically oscillating the above-mentioned porous silicon material support with a mixed solution of mass ratio of 40% hydrofluoric acid: 68% nitric acid: water=1:5:6, then blowing the porous silicon material support with nitrogen to dry, and placing it into a vapor deposition reaction chamber;
(2) vacuumizing the vapor deposition reaction chamber, and heating to 900∼1500°C under the protection of inert gas; and
(3) simultaneously introducing processing gas and hydrogen into the vapor deposition reaction chamber, and reacting for 2-3h at 900∼1500°C under a vacuum degree of 10∼15Pa; wherein the processing gas comprises tantalum metal compound and carrier gas, the tantalum metal compound is put in a source tank and heated to 200∼300°C, and the inert gas at 300°C as the carrier gas is introduced into the reaction chamber.

The preparation method further comprises a step of cooling the vapor deposition reaction chamber to 200°C under the protection of the inert gas after reaction in step (3).

In the above preparation method, the inert gas is preferably one of argon and helium or a mixture of the two, the most preferably argon.

In the above preparation method, a flow rate of the processing gas is preferably 80∼100ml/min.

In the above preparation method, a flow rate of the hydrogen is preferably 100∼150ml/min.

The porous tantalum metal material prepared by the method of the present invention has high porosity of greater than 85% and elastic modulus of less than 30Gpa, is similar to the mechanical properties of the human skeleton, and can be used as a permanent implant material applied in weight-bearing bone or non-weight-bearing parts in human body, such as hip and knee, skull, interbody fusion cage and so on. In addition, the tantalum metal coating of the porous tantalum metal material prepared by the method of the present invention is pyknotic and the thickness of the tantalum coating is 10∼50µm, which can also meet the hardness and the biocompatibility condition of the medical implant. The thickness of the tantalum coating can be 10∼15µm, which is beneficial to reducing the cost.

The medical porous tantalum metal material of the present invention adopts the high temperature resistant porous silicon material as a base material to construct a three-dimensional porous support similar to the skeleton microstructure. The support is in a three-dimensional reticular structure, which has the advantages of high porosity and connected porosity, light weight, moderate intensity, no cytotoxicity, good biocompatibility and so on.

The present invention has the advantageous effects that:
① The medical porous tantalum metal material of the present invention has high and uniform porosity, and is in a mutually communicated porous structure, has few pore dead space, is similar to human cancellous bone, and can promote bone ingrowth.
② The biocompatibility of the support material of the tantalum metal coating is good, and the best biomechanical properties are obtained according to the diameter of the coating and support pore. The adverse effects of stress shielding with the adjacent sclerotin after being implanted in vivo are reduced.
③ The preparation method of the present invention is relatively simple, and the porous silicon carbide of porous silicon material has high biological strength, small density, high strength, corrosion resistance and other unique properties, and is easy to be processed in a irregular shape suitable for the implanted site before tantalum plating, which avoids problems that the porous tantalum material is difficult to process, and the material is damaged greatly during processing, and the pore is damaged. At the same time, the tantalum metal coating is compacted with particle size of 1∼5µm and purity of over 99.5%, and has a sufficient thickness by controlling parameters such as reaction gas concentration, deposition temperature and reaction time during the chemical vapor deposition process, so as to obtain the sufficient mechanical strength of the material.
④ The porous tantalum metal material of the present invention can be applied to bone defect repair after bone trauma and osteonecrosis of multiple portions in the human body.

### Brief Description of the Drawings

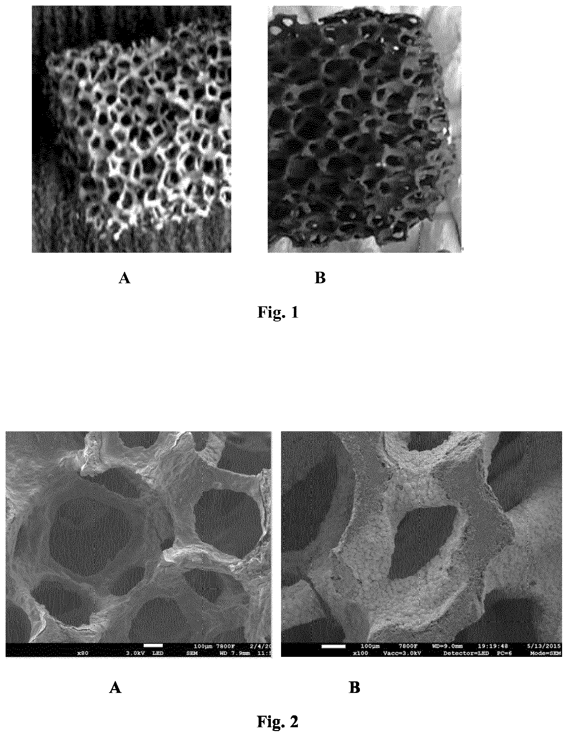

Fig. 1 shows the SEM drawings of a porous silicon carbide support before and after applying tantalum coating, wherein Fig. 1A is the SEM drawing of the porous silicon carbide support before applying the tantalum coating, and Fig. 1B is the SEM drawing of the porous silicon carbide support after applying the tantalum coating; and
Fig. 2 is SEM drawings of surface appearance of the porous silicon carbide support before and after applying the tantalum metal coating, wherein Fig. 2A is the SEM drawing of the surface appearance of the porous silicon carbide support before applying the tantalum metal coating, Figure 2B is the SEM drawing of the surface appearance of the porous silicon carbide support after applying the tantalum metal coating.

### Detailed Description of the Preferred Embodiments

The following nonrestrictive examples can enable those ordinary persons skilled in the art to comprehensively understand the present invention, without limiting the present invention in any way. Unless otherwise indicated in the following examples, experimental methods used are conventional methods, and reagents or the like used can be purchased from Chemical Agent Company. The porous silicon carbide and other porous silicon materials are commercial products, and can be purchased on the market.

### Embodiment 1

A preparation method for the medical porous tantalum metal material comprises the following steps:
(1) the porous silicon material support with the porosity of 85% and the pore diameter of 500µm is ultrasonically oscillated with a mixed solution of 40% hydrofluoric acid: 68% nitric acid: water = 1: 5: 6, then the porous silicon material support is blown with nitrogen to dry, and placed into a vapor deposition reaction chamber.
(2) intake/exhaust passage devices are connected, a sealing situation of the reaction chamber is checked, and the reaction chamber is vacuumized, and the reaction chamber is heated to 950°C under the protection of argon.
(3) The tantalum pentachloride powder with a diameter of 400-mesh is put into a source tank and heated to 200°C, and introduced into the reaction chamber with high temperature argon (300°C) as a carrier gas. A flow rate of the carrier gas is 120ml/min, a temperature of the reaction chamber is 950°C, and a vacuum degree of the reaction chamber is 10Pa. Hydrogen is introduced at a flow rate of 120ml/min while the argon is used as the carrier gas to introduce the tantalum pentachloride powder into the reaction chamber, and the reduction reaction is carried out for 2 hours, so that the tantalum metal reduced to metal powder is uniformly deposited on the surface of the porous silicon carbon support and the gap thereof, to obtain the medical porous tantalum metal material of the present invention.

The medical porous tantalum metal material prepared in embodiment 1 has a porosity of 85%, an elastic modulus of 10GPa, and a maximum compressive strength of 35MPa, wherein the porosity is detected according to the method in national standard GB/T 21650.1-2008, and the elastic modulus is detected according to the method in national standard GB/T 22315-2008.

Fig. 1 and Fig. 2 are SEM (Scanning Electron Microscope) drawings of the above-mentioned medical porous tantalum implant material, wherein Fig. 1A is the SEM drawing of the porous silicon carbide support before applying the tantalum coating, and Fig. 1B is the SEM drawing of the porous silicon carbide support after applying the tantalum coating. It can be seen that the surface of the porous silicon carbide is attached with dark black tantalum metal, so that the light reflection is weaken significantly; Fig. 2A and Fig. 2B show the surface appearances of the porous silicon carbide support before and after applying the tantalum coating at high magnification respectively, it can be seen that the surface of the porous silicon carbide support before applying the tantalum coating (Fig. 2A) has bright color and luster, so that stronger light reflection is shown in a digital microscopic image, which is not beneficial to the clear display of microstructure; while the light reflection of the surface of the porous silicon carbide after applying the coating (Fig. 2B) is weaken significantly, the microstructure of the porous support and the morphological characteristics of the pores are observed clearly, which shows good three-dimensional connectivity, and at the same time, the uneven surface of the tantalum coating is not only beneficial to cell adhesion and tissue embedding, but also enhance the linking strength of the support and bone tissue.

### Embodiment 2

A preparation method for the medical porous tantalum metal material comprises the following steps:
(1) the porous silicon material support having the porosity of 85% and the pore diameter of 400µm is ultrasonically oscillated with a mixed solution of 40% hydrofluoric acid: 68% nitric acid: water=1:5:6, then porous silicon material support is blown with nitrogen to dry, and placed into a vapor deposition reaction chamber.
(2) intake/exhaust passage devices are connected, a sealing situation of the reaction chamber is checked, and the reaction chamber is vacuumized, and the reaction chamber is heated to 1050°C under the protection of argon.
(3) The carbon pentachloride powder with a diameter of 400-mesh is put into a source tank and heated to 250°C, and introduced into the reaction chamber with high temperature argon (300°C) as a carrier gas. A flow rate of the carrier gas is 120ml/min, a temperature of the reaction chamber is 1050°C, and a vacuum degree of the reaction chamber is 15Pa. Hydrogen is introduced at a flow rate of 120ml/min while the argon is used as the carrier gas to introduce the tantalum pentachloride powder into the reaction chamber, and the reduction reaction is carried out for 2.5 hours, so that the tantalum metal reduced to metal powder is uniformly deposited on the surface of the porous silicon carbon support and the gap thereof, to obtain the medical porous tantalum metal material of the present invention.

The medical porous tantalum metal material prepared in embodiment 2 has a porosity of 85%, an elastic modulus of 12GPa, and a maximum compressive strength of 50MPa, wherein the porosity is detected according to the method in national standard GB/T 21650.1-2008, and the elastic modulus is detected according to the method in national standard GB/T 22315-2008.

## Claims

1. A medical porous tantalum metal material, wherein the porous tentalum metal material is prepared by means of a chemical vapor deposition method, in which a tantalum metal compound is reduced to tantalum metal powder, wherein the tantalum metal powder is evenly deposited on the surface of a porous silicon material support to form a tantalum coating;
wherein the tantalum metal compound is one of tantalum pentachloride and tantalum fluoride;
wherein the porosity of the porous silicon material is higher than 70%, the pore diameter of the porous silicon material being 100µm to 600µm;
wherein the thickness of the tantalum coating is 10µm to 50µm; **characterized in that**
the porous silicon is porous silicon carbide; and
the porosity of the tantalum metal material is higher than 85% and the elastic modulus thereof is less than 30Gpa.

2. The medical porous tantalum metal material according to claim 1, wherein the tantalum metal compound comprises powder with a particle size of 400-mesh.

3. A preparation method for the medical porous tantalum metal material according to claim 1, comprising the following steps of:
(1) ultrasonically oscillating the porous silicon material support according to claim 1 with a mixed solution of mass ratio of 40% hydrofluoric acid: 68% nitric acid: water=1:5:6, then blowing the porous silicon material support with nitrogen to dry and placing it into a vapor deposition reaction chamber;
(2) vacuumizing the vapor deposition reaction chamber, and heating to 900∼1500°C under the protection of inert gas; and
(3) simultaneously introducing processing gas and hydrogen into the vapor deposition reaction chamber, and reacting for 2-3h at 900∼1500°C under a vacuum degree of 10∼15 Pa; wherein the processing gas comprises tantalum metal compound and carrier gas, the tantalum metal compound is put in a source tank and heated to 200∼300°C, and the inert gas at 300°C as the carrier gas is introduced into the reaction chamber.

4. The method according to claim 3, wherein the inert gas is one of argon and helium or a mixture of the two.

5. The method according to claim 3, wherein a flow rate of the processing gas is 80∼100 ml/min.

6. The method according to claim 3, wherein a flow rate of the hydrogen is 100∼150 ml/min.

## Patentansprüche

1. Medizinisches poröses Tantal-Metallmaterial, wobei das poröse Tantal-Metallmaterial mittels eines chemischen Gasphasenabscheidungsverfahrens hergestellt wird, bei dem eine Tantal-Metallverbindung zu Tantal-Metallpulver reduziert wird, wobei das Tantal-Metallpulver gleichmäßig auf der Oberfläche eines porösen Silizium-Materialträgers abgeschieden wird, um eine Tantal-Beschichtung zu bilden;
wobei die Tantalmetallverbindung entweder Tantalpentachlorid oder Tantalfluorid ist;
wobei die Porosität des porösen Siliziummaterials mehr als 70% beträgt und der Porendurchmesser des porösen Siliziummaterials 100µm bis 600µm beträgt;
wobei die Dicke der Tantalbeschichtung 10µm bis 50µm beträgt;
**dadurch gekennzeichnet, dass**
das poröse Silizium poröses Siliziumkarbid ist; und
die Porosität des Tantal-Metallmaterials höher als 85 % ist und sein Elastizitätsmodul weniger als 30 GPa beträgt.

2. Medizinisches poröses Tantal-Metallmaterial nach Anspruch 1, wobei die Tantal-Metallverbindung Pulver mit einer Teilchengröße von 400 Mesh umfasst.

3. Verfahren zur Herstellung des medizinischen porösen Tantal-Metallmaterials nach Anspruch 1, umfassend die folgenden Schritte:
(1) Ultraschallschwingung des porösen Siliziummaterialträgers nach Anspruch 1 mit einer gemischten Lösung des Massenverhältnisses von 40% Flusssäure: 68% Salpetersäure: Wasser=1:5:6, dann Blasen des porösen Siliziummaterialträgers mit Stickstoff zum Trocknen und Einbringen in eine Reaktionskammer für die Dampfabscheidung;
(2) Vakuumieren der Reaktionskammer für die Dampfabscheidung und Erhitzen auf 900∼1500°C unter dem Schutz von Inertgas; und
(3) gleichzeitiges Einführen von Prozessgas und Wasserstoff in die Reaktionskammer für die Dampfabscheidung und Reagieren für 2 bis 3 Stunden bei 900 bis 1500°C unter einem Vakuumgrad von 10 bis 15 Pa; wobei das Prozessgas eine Tantal-Metallverbindung und ein Trägergas umfasst, die Tantal-Metallverbindung in einen Quellentank gegeben und auf 200 bis 300°C erhitzt wird, und das Inertgas bei 300°C als Trägergas in die Reaktionskammer eingeführt wird.

4. Verfahren nach Anspruch 3, wobei das Inertgas entweder Argon oder Helium oder ein Gemisch der beiden ist.

5. Verfahren nach Anspruch 3, wobei die Durchflussrate des Prozessgases 80-100 ml/min beträgt.

6. Verfahren nach Anspruch 3, wobei die Strömungsgeschwindigkeit des Wasserstoffs 100 bis 150 ml/min beträgt.

## Revendications

1. Matériau médical poreux en métal de tantale, dans lequel le matériau poreux en métal de tantale est préparé au moyen d'un procédé de dépôt chimique en phase vapeur, dans lequel un composé métallique de tantale est réduit en poudre de métal de tantale, dans lequel la poudre de métal de tantale est déposée uniformément sur la surface d'un support poreux en matériau de silicium pour former un revêtement de tantale ;
dans lequel le composé métallique de tantale est l'un des pentachlorure de tantale et fluorure de tantale ;
dans lequel la porosité du matériau de silicium poreux est supérieure à 70 %, le diamètre des pores du matériau de silicium poreux étant de 100µm à 600µm ;
dans lequel l'épaisseur du revêtement de tantale est de 10µm à 50µm ; **caractérisé en ce que**
le silicium poreux est du carbure de silicium poreux ; et que
la porosité du matériau métallique en tantale est supérieure à 85% et son module d'élasticité est inférieur à 30Gpa.

2. Matériau médical poreux en tantale métallique selon la revendication 1, dans lequel le composé en tantale métallique comprend une poudre avec une taille de particule de 400 mesh.

3. Procédé de préparation du matériau métallique poreux en tantale médical selon la revendication 1, comprenant les étapes suivantes consistant à :
(1) faire osciller par ultrasons le support en matériau de silicium poreux selon la revendication 1 avec une solution mixte de rapport massique de 40% d'acide fluorhydrique : 68% d'acide nitrique : eau=1:5:6, puis souffler le support en matériau de silicium poreux avec de l'azote pour le sécher et le placer dans une chambre de réaction de dépôt en phase vapeur ;
(2) mettre sous vide la chambre de réaction de dépôt en phase vapeur, et chauffer à 900∼1500°C sous la protection d'un gaz inerte ; et
(3) introduire simultanément un gaz de traitement et de l'hydrogène dans la chambre de réaction de dépôt en phase vapeur, et faire réagir pendant 2 à 3 heures à 900∼1500°C sous un degré de vide de 10∼15 Pa ; dans lequel le gaz de traitement comprend un composé métallique de tantale et un gaz porteur, le composé métallique de tantale est placé dans un réservoir source et chauffé à 200∼300°C, et le gaz inerte à 300°C en tant que gaz porteur est introduit dans la chambre de réaction.

4. Procédé selon la revendication 3, dans lequel le gaz inerte est l'un parmi l'argon et l'hélium ou un mélange des deux.

5. Le procédé selon la revendication 3, dans lequel un débit du gaz de traitement est de 80∼100 ml/min.

6. Procédé selon la revendication 3, dans lequel un débit de l'hydrogène est de 100∼150 ml/min.
